# EUROPEAN PATENT APPLICATION

(11) **EP 1 435 241 A1**
(43) Date of publication of application: **07.07.2004**
(21) Application number: 02029070.6
(22) Date of filing: 30.12.2002
(51) Int. Cl.: A61K 35/14, A61K 35/16, A01N 1/02, G01N 1/28, G01N 33/96

(54) **Method for stabilizing blood, serum, or plasma specimen and container containing ph buffer agent**

(71) Applicant: Kyokuto Pharmaceutical Industrial Co. Ltd, Chuo-Ku, Tokyo 103-0024 (JP)
(72) Inventor: Arima, Kazuhisa, c/o 3333-26 Aza Asayama, Ibaraki 318-0004 (JP); Kojima, Masaharu, c/o 3333-26 Aza Asayama, Ibaraki 318-0004 (JP); Nakano, Shinya, c/o 3333-26 Aza Asayama, Ibaraki 318-0004 (JP); Gunji, Tomoko, c/o 3333-26 Aza Asayama, Ibaraki 318-0004 (JP)
(74) Representative: Grünecker, Kinkeldey, Stockmair & Schwanhäusser Anwaltssozietät

(57) **Abstract**

The object of the present invention is to provide convenient methods that enable proper analyses of blood components by stabilizing those components, and a container that is useful for the methods.

The object is attained by a method for stabilizing a blood, serum, or plasma specimen comprising 1-a) adding a pH buffer agent to a blood specimen, 1-b) preparing a serum or plasma specimen and adding a pH buffer agent to the specimen, or 1-c) putting a blood, serum, or plasma specimen into a container that contains a pH buffer agent, and 2) storing the specimen obtained in step 1-a, 1-b, or 1-c, wherein the pH buffer agent has an initial pH in the range of a weakly acidic pH to a slightly basic pH and has a buffer ability that maintains the pH of the specimen obtained in step 1-a, 1-b, or 1-c around neutral in step 2.

The object is also attained by using a container that a blood, serum, or plasma specimen contacts, containing a pH buffer agent having an initial pH in the range of a weakly acidic pH to a slightly basic pH and a buffer ability that maintains the pH of the specimen around neutral.

The object is attained by another method for stabilizing a serum or plasma specimen comprising 1-a) adding a pH buffer agent to a blood specimen, or 1-b) putting a blood specimen into a container that contains a pH buffer agent, 2) dissolving the pH buffer agent in the blood specimen, 3) preparing a serum or plasma specimen from the specimen obtained in step 2, and 4) storing the serum or plasma specimen obtained in step 3, wherein the pH buffer agent has an initial pH in the range of a weakly acidic pH to a slightly basic pH and has a buffer ability that maintains the pH of the serum or plasma specimen obtained in step 3 around neutral in step 4.

## Description

### Technical field:

The present invention relates to a method for stabilizing a blood, serum, or plasma specimen, in other words, a method for preventing the deterioration of components in blood, serum, or plasma, and a container that is useful for the method. The present invention is useful in fields of clinical diagnostic tests, clinical research, and basic research.

### Background Art:

Analyses of blood components are widely conducted in various clinical diagnostic tests, clinical research, and basic research. Commonly, serum or plasma is prepared from blood of a subject and various components contained in it are analyzed. In these analyses it is important that those various components remain stable until they are analyzed and that the results of the analyses properly reflect the conditions of the subject in the body.

European Patent Publication No. 0359201 discloses a method for stabilizing blood coagulation (or clotting) factors. In this method, glycylglycine or glycylglycylglycine, or both, is added to blood or plasma. Japanese Patent Early-publication 8-224227 discloses a blood-collecting tube comprising an aminocarboxylic acid and a Good's buffer that are enclosed in the tube. The aminocarboxylic acid and Good's buffer contribute to stabilize blood clotting factors. Thus, methods for stabilizing blood clotting factors for clinical diagnostic tests have been proposed. However, no method has been known, by which method all or most blood components are stabilized. Also, no method has been known, by which method all or most, serum or plasma components are stabilized.

Under the situation described above, fresh blood, serum, or plasma specimens have been commonly analyzed. If it is necessary to store blood, serum, or plasma for a short period of time, it is refrigerated at 4°C. If it is necessary to store blood for a longer period of time, the blood is frozen immediately after sampling of it, transported, and thawed just before use for analyses. If it is necessary to store serum or plasma for a longer period of time, the serum or plasma is frozen immediately after preparing it from blood, transported, and thawed just before use for analyses.

Although refrigeration at 4°C is convenient, it is commonly believed that serum or plasma degrade in a long period of time even though it is stored at 4°C. Also in catalogues of clinical reference laboratories, for most test items it is written that specimens are required or recommended to be frozen. For only a few of those test items, it is written that storing specimens at a low temperature (4°C) or room temperatures (ambient temperatures) is accepted.

Freezing blood immediately after its sampling or freezing serum or plasma immediately after its preparation, and keeping it in a frozen state until its use require that the temperatures during its freezing and storing are controlled very carefully. Also, the temperature of serum or plasma should be precisely controlled after thawing it by, e.g., putting it on ice or should be analyzed immediately after thawing it. Thus, this means are inconvenient.

In recent years, systems in which specimens are sent to clinical reference laboratories by mail for analyses have also been planned for some test items. In such systems, it is very difficult to keep the specimens at a low temperature or in a frozen state. Thus, in such systems specimens may deteriorate during transportation. This is because the specimens may be left at room temperatures for a long period of time and/or a cold insulator, which has been put near the specimens, may get warm, and thus the temperatures of the specimens may rise. This is problematic.

The object of the present invention is to provide a convenient method that enables proper analyses of blood components by stabilizing those components, and a container that is useful for the method.

### Summary of the Invention:

The present inventors have found that blood, serum, or plasma specimens as objects for clinical diagnostic tests or research materials had neutral pHs (i.e., pH values) immediately after sampling blood or preparing serum or plasma due to reflecting the conditions in the organisms and that, however, their pH values surprisingly rose to reach alkaline pHs after they had been stored for a long period of time, left at room temperatures, or heated. More specifically, the pH values of the specimens were initially around pH 7.4, and rapidly rose to be alkaline pHs of around pH 9. Further, the pH markedly rose when the volume of the specimen was small.

The present inventors examined pH changes of a fresh serum specimen (100 µl) by incubating it at 4°C, room temperatures, 30°C, or 37°C, and have found that the pHs (i.e., pH values) shifted to alkaline pHs within several hours. This pH shifting was remarkable when the specimen had been heated. In fact, the pH reached almost pH 9 within one hour by incubating it at 37°C. Even when it had been left at room temperatures, the pH reached about pH 8.5 within one hour and pH 9 within three or four hours. When a 100 µl- sample of the specimen had been stored at 4°C or when a 1 ml-sample of it had been left at room temperatures, the pH of the sample rose more moderately. However, the pH of the sample reached around pH 8.5 within four hours.

The pH of a blood, serum, or plasma specimen basically rises with a lapse of time. Therefore, it is inevitable that blood components which are not stable under alkaline conditions degrade with a lapse of time. The pH is liable to shift from neutral to alkaline particularly when a specimen is heated. Moreover, when the specimen has an alkaline pH and is heated, the degradation is enhanced as compared to the case where the specimen is kept cool. Recently the volume of specimen, which clinical reference laboratories and the like use for analyses, is becoming smaller. However, the present inventors have understood that the pH of the specimen easily rise during storage or after thawing when its volume is small. The pH rising after thawing occurs even if reagents that are used to analyze blood components are buffered in an adequate range. Therefore, the test results must be affected by the degradation of the blood components that has been resulted from the pH rising prior to analyses.

The present inventors thought that the stability of a blood, serum or plasma specimen would be enhanced by preventing rising the pH of the specimen, and conducted some experiments. As a result, they have found that the stability was indeed enhanced by mixing a pH buffer agent with the blood, serum, or plasma specimen. The present invention has been attained based on this finding.

Namely, the present invention provides, as the first method, a method for stabilizing a blood, serum, or plasma specimen comprising:
1-a) adding a pH buffer agent to a blood specimen,
1-b) preparing a serum or plasma specimen and adding a pH buffer agent to the specimen, or
1-c) putting a blood, serum, or plasma specimen into a container that contains a pH buffer agent, and
2) storing the specimen obtained in step 1-a, 1-b, or 1-c,
wherein the pH buffer agent has an initial pH in the range of a weakly acidic pH to a slightly basic pH and has a buffer ability that maintains the pH of the specimen obtained in step 1-a, 1-b, or 1-c around neutral in step 2.

The present invention according to the above method may include the following embodiments, singly or in combination of two or more of them:
i) the above method wherein the pH buffer agent maintains the pH of the specimen within the range of pH 7.0 to pH 8.0 in step 2;
ii) the above method wherein the pH buffer agent has an initial pH within the range of pH 5.5 to pH 8.0;
iii) the above method wherein the pH buffer agent has a form selected from the group consisting of a solid, powder, granules, coating, a droplet, and microdroplets;
iv) the above method wherein the pH buffer agent has been prepared by vacuum drying (including drying under reduced pressures and freeze drying), heat transfer drying (including convective heat transfer drying, radiation heat transfer drying, and conduction heat transfer drying), internal exothermic drying, drying by using a desiccant, ultrasonic drying, or air drying an aqueous solution of the pH buffer agent that has been contained in the container; and
v) the above method wherein the container is a vacuum or non-vacuum blood collection tube, a centrifuge tube for separating serum or plasma, a urine collection cup, a container for storing a specimen, a 96-well microtiter plate or a module thereof, a 384-well plate, or a specimen container that is used when the specimen is sent to a clinical reference laboratory by mail.

Further, the present invention provides a container that a blood, serum, or plasma specimen contacts, containing a pH buffer agent having an initial pH in the range of a weakly acidic pH to a slightly basic pH and a buffer ability that maintains the pH of the specimen around neutral.

The present invention according to the above container may include the following embodiments, singly or in combination of two or more of them:
i) the above container wherein the pH buffer agent maintains the pH of the specimen within the range of pH 7.0 to pH 8.0;
ii) the above container wherein the pH buffer agent has an initial pH within the range of pH 5.5 to pH 8.0;
iii) the above container wherein the pH buffer agent has a form selected from the group consisting of a solid, powder, granules, coating, a droplet, and microdroplets;
iv) the above container wherein the pH buffer agent has been prepared by vacuum drying (including drying under reduced pressures and freeze drying), heat transfer drying (including convective heat transfer drying, radiation heat transfer drying, and conduction heat transfer drying), internal exothermic drying, drying by using a desiccant, ultrasonic drying, or air drying an aqueous solution of the pH buffer agent that has been contained in the container; and
v) the container is a vacuum or non-vacuum blood collection tube, a centrifuge tube for separating serum or plasma, a urine collection cup, a container for storing a specimen, a 96-well microtiter plate or a module thereof, a 384-well plate, or a specimen container that is used when the specimen is sent to a clinical reference laboratory by mail.

Furthermore, the present invention provides, as the second method, a method for stabilizing a serum or plasma specimen comprising:
1-a) adding a pH buffer agent to a blood specimen, or
1-b) putting a blood specimen into a container that contains a pH buffer agent,
2) dissolving the pH buffer agent in the blood specimen,
3) preparing a serum or plasma specimen from the specimen obtained in step 2, and
4) storing the serum or plasma specimen obtained in step 3,
wherein the pH buffer agent has an initial pH in the range of a weakly acidic pH to a slightly basic pH and has a buffer ability that maintains the pH of the serum or plasma specimen obtained in step 3 around neutral in step 4.

### Detailed Description of the Invention:

Hereafter, the present invention will be specifically explained.

The methods and container of the present invention are characterized in that a blood, serum, or plasma specimen contacts a pH buffer agent having an initial pH in the range of a weakly acidic pH to a slightly basic pH and a buffer ability that maintains the pH of the specimen around neutral.

In the methods of the present invention, the pH of a blood, serum, or plasma specimen is controlled to be around neutral by the co-existence of the pH buffer agent. Thereby the deterioration of components that are not always stable under alkaline conditions with a lapse of time can be highly reduced. The methods can be conducted by using the container of the present invention. Namely, the pH of a blood, serum, or plasma specimen can be easily controlled by placing, in advance, the pH buffer agent or a buffer solution derived from the agent inside a specimen container such as a blood collection tube.

A "pH buffer agent" and a ''buffer agent" have a same meaning and refer to the effective ingredient(s) that can show a pH buffer action. A ''buffer solution" is an aqueous solution in which the pH buffer agent has been dissolved. The buffer solution may contain other ingredients such as saccharides, polymeric substances, chemical substance, and biological substances in addition to the buffer agent. The pH of the buffer solution can be adjusted with an acid (e.g., HCl) or an alkali (e.g., NaOH or KOH), or by mixing at an appropriate ratio compounds that show different pH values, e.g., monosodium salt and disodium salt of a polybasic acid. An "initial pH" means the pH of an aqueous solution of the buffer agent. Namely, it is the pH that is indicated by the aqueous solution of the buffer agent immediately after the solution was prepared or the pH of the solution was adjusted by using an acid or an alkali. An "alkaline" and a ''basic'' have a same meaning and refer to a pH of above 7.0. The terms "around neutral" means herein from about pH 6.5 to pH 8.

Examples of the pH buffer agents that can be used in the present invention include, but not limited to them, phosphate buffers, ACES [N-(2-acetamido)-2-aminoethanesulfonic acid], ADA [N-(2-acetamido)-2-iminodiacetic acid], BES [N,N-bis(2-hydroxyethyl)-2-aminoethanesulfonic acid], BICINE [N,N-bis(2-hydroxyethyl)glycine], BIS-TRIS [bis(2-hydroxyethyl)-. imino-tris(hydroxymethyl)methane], BIS-TRIS-PROPANE [1,3-bis[tris-(hydroxymethyl)methylamino]propane], DIPSO [3-[N,N-bis(2-hydroxyethyl)-amino]-2-hydroxypropanesulfonic acid], EPPS [N-(2-hydroxyethyl)piperazine-N'-(3-propanesulfonic acid); HEPPS], HEPES [N-(2-hydroxyethyl)piperazine-N'-(2-ethanesulfonic acid)], HEPPESO [N-(2-hydroxyethyl)piperazine-N'-(2-hydroxypropanesulfonic acid)], IMIDAZOLE [1,3-diaza-2,4-cyclopentadiene], MES [2-(N-morpholino)ethanesulfonic acid], MOBS [4-(N-morpholino)-butanesulfonic acid], MOPS [3-(N-morpholino)-propanesulfonic acid], MOPSO [3-(N-morpholino)-2-hydroxypropanesulfonic acid], PIPES [piperazine-N, N'-bis(2-ethanesulfonic acid)], POPSO [piperazine-N, N'-bis(2-hydroxypropanesulfonic acid)], TAPS [N-tris(hydroxymethyl)methyl-3-aminopropanesulfonic acid], TAPSO [3-[N-tris(hydroxymethyl)methylamino]-2-hydroxypropanesulfonic acid], TEA [triethanolamine], TES [N-tris(hydroxymethyl)methyl-2-aminoethanesulfonic acid], TRICINE [N-tris(hydroxymethyl)methylglycine], TRIS [tris(hydroxymethyl)aminoethane], glycylglycine, acetate buffers, and citrate buffers.

Buffer agents, other than those listed above, having strong buffer abilities around pH 6.0 to pH 8.0 may also be suitably used. The terms ''buffer ability" means the pH range that a buffer agent can show buffer action when it has been dissolved in a predetermined amount of an aqueous solution. In some special cases, for example, in the case where it is aimed that only specific components in a specimen should not be degraded and in the case where the amount of the buffer solution can be increased or decreased, buffer agents that have a strong buffer abilities at or above pH 8.1 or at or below pH 5.9 may be used. Thus, buffer agents are not limited to those listed above, and may be appropriately selected by those skilled in the art.

In the methods or container of the present invention, these buffer agents or buffer solutions can be used independently or in combination of two or more of them.

The pH range within which the blood, serum, or plasma specimen should be controlled by the pH buffer agent is around neutral, i.e., from about pH 6.5 to pH 8, preferably from pH 6.8 to pH 8.0, more preferably from pH 7.0 to pH 8.0, and still more preferably from pH 7.1 to pH 7.8.

To control the pH within the above range (i.e., pH 6.5 to pH 8), a buffer agent having an initial pH in the range of a weakly acidic pH to a slightly basic pH is used. The buffer agent has an initial pH of preferably 5.5 to 8.0, more preferably 6.0 to 7.7, and still more preferably 6.5 to 7.5. The reasons are as follows.

The pH of the specimen rises to around pH 9 with a lapse of time. To prevent the specimen from rising its pH above pH 8.0, a buffer agent must be used in a high concentration if the initial pH of the buffer agent is near and below pH 8.0. On the other hand, if the initial pH of it is below pH 5.5, the final pH of the specimen can be readily controlled around neutral. However, when the volume of a fresh specimen is smaller than a volume that is expected for a container, the specimen having a neutral pH may be exposed to excessively acidic pH immediately after it contacts the buffer agent. Therefore, the buffer agent has suitably an initial pH of 5.5 to 8.0, and preferably 6.0 to 7.7. To avoid a transient pH lowering that occurs upon mixing a fresh specimen with a buffer agent (or buffer solution) and to avoid large increase of pH during storage, buffer agents having an initial pH within the range of pH 6.5 to 7.5 can be suitably used. However, it should be noted that buffer agents having an initial pH of 6.0 to 6.5 may also be used. This is because if a small amount of the buffer agent is used, the pH of the specimen may be controlled within the targeted range.

When a buffer agent is selected, effects of other substances, for example, anti-coagulants such as sodium citrate and coagulation accelerants, should be considered.

Further, whether the pH of a specimen can be controlled within a targeted pH range also depends on 1) a pH range where a specific buffer agent to be used has a strong buffer ability or buffering potential, and 2) the concentration of the buffer agent in an aqueous solution that comprises a blood, serum, or plasma specimen. It cannot be determined only by the initial pH of the specific buffer agent. What is important is to control the pH of a specimen around neutral. To achieve that goal, those skilled in the art can appropriately select the type and amount of a buffer agent, the initial pH, and the like. However, the buffer agent is commonly used in such an amount that its concentration in the aqueous solution becomes preferably from 0.01 to 0.3M, more preferably from 0.03 to 0.1M.

A buffer agent with essentially no aqueous components is preferably used. Namely, it is preferable that aqueous components are essentially devoid. This is because a blood, serum, or plasma specimen is diluted if a large amount of aqueous components exist (i.e., a buffer solution having a low concentration is used), and, as a result, concentrations of components contained in the specimen become low. Thus, a preferable example of methods for placing, in advance, a buffer agent into a container comprises removing water from a buffer solution contained in the container to form a solid, powder, granules, or coating. Examples of methods for drying the buffer solution include vacuum drying (including drying under reduced pressures and freeze drying), heat transfer drying (including convective heat transfer drying, radiation heat transfer drying, and conduction heat transfer drying), internal exothermic drying, drying by using a desiccant, ultrasonic drying, air drying, and combinations of two or more of them. Alternatively, a buffer solution having a very small volume that can be ignored in an analysis may be placed in a container, or a droplet or microdroplets of a buffer solution may be attached to the inner wall of a container. It is preferable that a buffer agent having a form of a solid, powder, or granules (particularly powder or granules), or a buffer solution having a form of a droplet or microdroplets is in advance prepared in a container.

In the present invention, the term "a container" refers comprehensively to an article that can contain or hold a blood, serum, or plasma specimen for sampling, storing, transportation, diagnostic tests, research, and the like, regardless whether it is a long or short period of time. Thus, examples of the "container" include a plate-like, block-like, or strip-like paper or sponge, a piece of plastic etc., in addition to various forms of glass or plastic tubes, cups, microtiter plates, and the like. More specifically, examples of the "container" include vacuum or non-vacuum blood collection tubes, centrifuge tubes for the serum or plasma separation, urine collection cups, sample storage containers, 96-well microtiter plates or their modules, 384-well plates, sample containers that are used to send specimens to clinical reference laboratories by mail, and the like. A module of a microtiter plate means a unit of one or more lines in a microtiter plate that has been constructed so that the unit can be removed (for example, a unit includes one or more lines wherein one line includes 8 or 12 wells in the case of a 96-well microtiter plate). There is a specimen container that is used when a specimen is sent to a clinical reference laboratory by mail. This specimen container is used in sampling, transporting, and storing a specimen. Although the form of the specimen container may vary depending on the components to be analyzed, the clinical reference laboratories, and the like, filter paper is often used.

To avoid spilling or flaking-off of the buffer agent that has been placed in a container, a closed-type container such as a vacuum blood collection tube is better from the viewpoint of handling.

In the first method of the present invention, in step 1, a) a pH buffer agent is added to a blood specimen, b) a serum or plasma specimen is prepared from blood and a pH buffer agent is added to the serum or plasma specimen, or c) a blood, serum, or plasma specimen is put into a container that contains a pH buffer agent. In the second method of the present invention, in step 1, a) a pH buffer agent is added to a blood specimen or b) a blood specimen is put into a container that contains a pH buffer agent. Namely, a buffer agent or a buffer solution may have been placed in a container before a blood, serum, or plasma specimen is poured into the container. Alternatively, it may be put into a container after the specimen has been poured into the container. In order to minimize the dilution of the specimen, a buffer agent having a form of powder or granules that has been formulated to show a certain range of pH when it is dissolved in a predetermined amount of aqueous solution is preferably used.

More specifically, at the time of sampling of blood, a buffer agent may be present in a container such as a blood collection tube. Alternatively, the buffer agent may be combined with a blood, serum, or plasma specimen after collecting the blood specimen or after preparing the serum or plasma specimen. In the latter case, blood is collected or gathered and then transferred to a container that contains a buffer agent. Or, blood is collected or gathered, serum or plasma is prepared from the blood, and the serum or plasma is poured into a container that contains a buffer agent. Or, the buffer agent may be directly added to the collected blood or the prepared serum or plasma. Ideally, however, it is preferred to collect the blood directly into a vacuum tube that contains a buffer agent in view of the reproducibility of conditions after sampling. This is because, if blood is first collected into a tube that does not contain the buffer agent, the serum or plasma is prepared from the blood at need, and then the buffer agent is added to the blood, serum, or plasma specimen or the specimen is transferred to a container that contains a buffer agent, the time between the blood-collecting and mixing the specimen with the buffer agent varies.

The time between the blood-collecting and mixing the specimen with the buffer agent is preferably one hour or less when the specimen has a volume of about 1 ml, is exposed to air, and is stored at 25°C. The time is preferably fifteen minutes or less when the specimen has a volume of about 0.1 ml, is exposed to air, and is stored at 25°C and is preferably thirty minutes or less when the specimen has a volume of about 0.1 ml, is exposed to air, and is stored at 4°C.

Some blood components contained in serum have been analyzed by using a serum specimen prepared as follow: Blood is collected or gathered into a vacuum blood collection tube and then centrifuged to separate into serum and a clot. The serum is immediately frozen. The frozen specimen is stored and then thawed. After thawing, the amount of a component(s) is determined. The standard value of the amount of the component is specified based on the values obtained by using the thawed serum specimen as described above. To compare with the standard value, the pH buffer agent may be added to a serum specimen immediately after the frozen serum specimen is thawed. Also for a blood or plasma specimen, this can be applied.

In the first method of the present invention, the specimen that also contains a buffer agent is stored until the specimen is analyzed. The storing condition is not limited. However, generally, it is stored for a short period of time in a refrigerator or at room temperatures and for a longer period of time in a freezer.

The term "storing" in step 2 of the first method of the present invention and in step 4 of the second method of the present invention means that a mixture of a pH buffer agent and a blood, serum, or plasma specimen is left a period of time. The period of time may be a very, very short time or a long time. For example, when the mixture is immediately subjected to a preparation (e.g., mixing with a reagent) for an analysis, the period of time is very, very short. Also in this case, the present invention is useful to prevent the deterioration of the component during its determination.

In the second method of the present invention, a pH buffer agent is dissolved in a blood specimen and then a serum or plasma specimen is prepared from the blood specimen. Dissolution of the pH buffer agent and the preparation of the serum or plasma specimen can be conducted by known methods. The serum or plasma specimen thus obtained is stored until the specimen is analyzed. The storing condition is not limited. However, generally, it is stored in a short period of time in a refrigerator or at room temperatures and a longer period of time in a freezer.

According to the methods of the present invention, it is possible to enhance the stability of a blood, serum, or plasma specimen with a lapse of time by preventing rising the pH of the specimen.

After collecting blood, the pH of a blood, serum, or plasma specimen increases with time and shifts from neutral to alkaline range. This pH shifting markedly proceed when the volume of the specimen is small. Among components in the specimen, those having a poor stability under alkaline conditions degrade reversibly or irreversibly, depending on the nature of the component and on the degree of pH increase. The degradation proceeds slower under low temperatures, whereas both pH increase and degradation of components are accelerated under elevated temperatures. In the methods and container of the present invention, pH increase is prevented by using a pH buffer agent. As a result, the degradation of components that are unstable under alkaline conditions in the blood, serum, or plasma specimen is prevented. Therefore, according to the present invention, constant assay results can be obtained. More specifically, the assay results obtained from a specimen that has been stored for a certain period of time are nearly the same as those obtained from a fresh specimen. Similarly, the assay results in the case where a specimen sample has been left under room temperatures during its diagnostic test or assay are nearly the same as those in the case where the specimen sample has been refrigerated. Thus, by the present invention, the stability of a blood, serum, or plasma specimen with a lapse of time can be enhanced, and, as a result, the reproducibility and reliability of clinical diagnostic tests as well as clinical and basic research experiments can be improved.

By using the first method, the second method, or the container of the present invention, the pH of a specimen is maintained around neutral, and therefore it becomes possible to protect certain types of components which are to be analyzed from degradation, not only when the specimen is left under room temperatures but also when it is heated to 37°C. Namely, the present invention can solve the problem of instability of specimens resulting from the specimens being exposed to various temperature conditions. Thus, the present invention contributes to realize a new examination system in which the specimens are sent to clinical reference laboratories by mail and analyzed in the laboratories.

The present invention provides an additional merit. Namely, the present invention exercises usefulness in developing diagnostic agents where blood, serum, and plasma are used as specimens. In developing a new diagnostic agent, a single specimen is repeatedly used to determine the assay conditions and also to examine sensitivity, specificity, reproducibility, correlation with the results obtained by a different method, effects of co-existent substances etc., and storage or preservation stabilities. In such a situation, the test results may become variable if the specimen is significantly degraded. The present invention prevents the degradation of specimens that is resulted from, at least, pH increase. Therefore, it can provide advantages in studies in which a specimen is repeatedly assayed.

The above advantages of the present invention are very important in developing an assay method using a protein chip (i.e., a microarray) and the like that have been actively investigated recently. This is because, unlike conducting a single analysis in which optimum conditions are selected for a single component to be analyzed, to try to conduct a multi-item assay method in which many components are determined at once using microarrays, etc., a set of conditions that is adaptable for all assays should be found. Therefore, a specimen is assayed in a large number of times. In such a situation, the present invention can significantly contribute to the acceleration of developing diagnostic agents and to the improvement of reliabilities of measurements, because it prevents the degradation of specimens.

### Examples:

Hereafter, the present invention will be specifically explained by referring to examples. However, it should not be understood that the present invention is restricted to these examples.

### Example 1: pH changes of serum specimen left in 96-well microtiter plates on which pH buffer agents had been solidified

Into each well of 96-well microtiter plates used as containers for specimens, 50 µl of 0.1 M phosphate buffer solution (pH 6.0) (hereafter "PB 6.0"), 0.1 M phosphate buffer solution (pH 6.5) (hereafter "PB 6.5"), 0.1 M HEPES buffer solution (pH 6.0) (hereafter "HEPES 6.0"), or 0.1 M HEPES buffer solution (pH 6.5) (hereafter "HEPES 6.5") was poured. The liquid components in the buffer solutions were removed by drying the plates in a desiccator to solidify the buffer agents on the surfaces of wells. Each of the thus-obtained plates is called as "a pH buffer agent-solidified plate." For comparison, 96-well microtiter plates (hereafter each "an untreated plate") and a 1.5-ml sample tube, where no buffer agent had been solidified, were also used.

Serum specimen was prepared as follows: Blood was gathered to a vacuum blood collection tube that contains a blood coagulation accelerator and a blood separating agent. After 30 minutes, the blood was centrifuged and serum was collected. The serum was subdivided and then frozen. By thawing the frozen serum, a serum specimen was obtained.

Into the specimen containers as prepared above, the serum specimen was poured in an amount of 100 µl/well of the microtiter plates or 1.0 ml/1.5-ml sample tube. The specimen containers were left under the temperature condition of 25°C. The concentrations of the buffer agents were 50 mM after pouring the serum into the wells of the PB-solidified plates and HEPES-solidified plates. Another untreated plate, into which wells the fresh serum specimen had been poured, was left at 4 °C. The pHs of the serum specimens and those of serum specimen/buffer agent mixtures were measured at 1, 2, 4, and 20 hours after the serum specimen had been poured. The results are shown in Table 1.

**Table 1:**

| Measurement of Serum's pH | | | | | | | | |
|---|---|---|---|---|---|---|---|---|
| Storage Condition | | | pH | | | | | |
| Container | amount | temp. | -* | 0hr | 1hr | 2hrs | 4hrs | 20hrs |
| Untreated Plate | 100 µl | 25°C | 7.8 | 7.8 | 8.6 | 8.7 | 8.9 | 9.2 |
| Untreated Plate | 100 µl | 4°C | 7.8 | 7.8 | 8.2 | 8.3 | 8.4 | 8.7 |
| Untreated 1.5-ml Sample Tube | 1.0ml | 25°C | 7.8 | 7.8 | 8.0 | 8.1 | 8.6 | 9.1 |
| PB 6.0-solidified Plate | 100 µl | 25°C | 7.8 | 7.0 | 7.1 | 7.1 | 7.1 | 7.1 |
| PB 6.5-solidified Plate | 100 µl | 25°C | 7.8 | 7.5 | 7.5 | 7.5 | 7.6 | 7.6 |
| HEPES 6.0-solidified Plate | 100 µl | 25°C | 7.8 | 7.1 | 7.2 | 7.5 | 7.6 | 7.6 |
| HEPES 6.5-solidified Plate | 100 µl | 25°C | 7.8 | 7.1 | 7.3 | 7.5 | 7.7 | 7.7 |

| | | | | | | | | |
|---|---|---|---|---|---|---|---|---|
| * pH of the serum before the experiment | | | | | | | | |

Large changes in pH values of the serum specimens were observed in the untreated plates and the sample tube used as controls. In particular, in the untreated plate left under the 25°C storage condition, the pH of the serum specimen elevated from 7.8 to 8.6 within one hour and reached pH 9.2 after 20 hours. The pH changes of the serum specimens in the untreated plate stored at 4°C and in the 1.5-ml sample tube were not as drastic as the case where the untreated plate was stored at 25°C. However, still in both cases the pHs reached about pH 9 after 20 hours. On the other hand, in the serum specimens that were poured into the pH buffer agent-solidified plates (i.e., the serum specimen/ buffer agent mixtures), the pH changes were smaller between immediately after pouring the serum specimens and 20 hours after. Specifically, the pHs slightly changed in PB 6.0-solidified plate from 7.0 to 7.1 and in PB 6.5-solidified plate from 7.5 to 7.6. Also, the pHs changed in HEPES 6.0-solidified plate from 7.1 to 7.6 and in HEPES 6.5-solidified plate from 7.1 to 7.7. The pHs of specimens that were poured into pH buffer agent-solidified plates were kept below pH 8 even after 20 hours.

### Example 2: Serum Pepsinogen I / II Assays in Serum Specimens in pH Buffer Agent-Solidified Plates

PB 6.0-solidified plates and HEPES 6.5-solidified plates were prepared in the same manner as described in Example 1, except that to solidify the buffer agents the plates were put in an incubator at 37°C for one day. Serum specimen was prepared in the same manner as described in Example 1.

One hundred microliter per well of the serum specimen were poured into wells of the PB 6.0-solidified plates, HEPES 6.5-solidified plates, and untreated plates as controls. The plates were left for four hours at 4°C (control), 25°C, 37°C, 43°C, and 50°C. Then, the amounts of pepsinogen I and pepsinogen II in the serum specimens were assayed by using a sandwich ELISA method.

More specifically, a prescribed amount of one of the serum specimens as prepared above was sampled. By using a prototype of ELUMINA PEPSINOGEN I / ELUMINA PEPSINOGEN II (Kyokuto Pharmaceutical Industrial Co.), the amounts of pepsinogen I and pepsinogen II in the serum specimen were respectively measured according to the reaction conditions recommended by the manufacturer. By an anti-human pepsinogen I monoclonal antibody, which was immobilized on a well of a 96-well microtiter plate, pepsinogen I was captured and detected with a mixture of an alkaline phosphatase-labeled anti-human pepsinogen I antibody and an alkaline phosphatase-labeled anti-human pepsinogen II antibody. By an anti-human pepsinogen II monoclonal antibody, which was immobilized on another well of the 96-well microtiter plate, pepsinogen II was captured and detected with the mixture. The results are shown in Tables 2 and 3.

**Table 2:**

| Assay of Serum Pepsinogen I | | | | | | |
|---|---|---|---|---|---|---|
| | | Storage Temperature | | | | |
| | | 4°C | 25°C | 37°C | 43°C | 50°C |
| Untreated Plate | measured value (ng/ml) | 54.4 | 46.8 | 4.9 | 1.9 | 1.7 |
| | residual content (%) | 100 | 86.0 | 9.0 | 3.4 | 3.1 |
| PB 6.0-solidified Plate | measured value (ng/ml) | 54.2 | 54.6 | 53.0 | 50.1 | 11.7 |
| | residual content (%) | 100 | 100 | 97.8 | 92.4 | 21.7 |
| HEPES 6.5-solidified Plate | measured value (ng/ml) | 56.3 | 53.9 | 51.5 | 26.4 | 1.8 |
| | residual content (%) | 100 | 95.7 | 91.5 | 46.9 | 3.2 |

**Table 3:**

| Assay of Serum Pepsinogen II | | | | | | |
|---|---|---|---|---|---|---|
| | | Storage Temperature | | | | |
| | | 4°C | 25°C | 37°C | 43°C | 50°C |
| Untreated Plate | measured value (ng/ml) | 12.5 | 11.7 | 11.1 | 6.3 | 1.6 |
| | residual content (%) | 100 | 93.6 | 88.8 | 50.4 | 12.8 |
| PB 6.0-solidified Plate | measured value (ng/ml) | 12.5 | 12.8 | 12.7 | 11.6 | 6.8 |
| | residual content (%) | 100 | 102 | 102 | 92.8 | 54.4 |
| HEPES 6.5-solidified Plate | measured value (ng/ml) | 13.2 | 13.6 | 13.0 | 12.0 | 2.8 |
| | residual content (%) | 100 | 103 | 985 | 90.9 | 21.2 |

In the assay of pepsinogen I, when the untreated plates were used, a tendency was showed that the measured value lowered at 25°C and the measured value markedly dropped at 37°C. At this temperature, the residual content (i.e., the percentage of each measured value based on the measured value of the serum specimen stored at 4°C) was only 9.0%. On the other hand, when the PB 6.0-solidified plates were used, no fluctuation of the measured values was seen up to 43 °C. Namely, the residual contents were 97.8% at 37°C and 92.4% at 43°C, respectively. Similarly, when the HEPES 6.5-solidified plates were used, the residual content was 91.5% at 37 °C.

In the assay of pepsinogen II, the effects of the temperature were smaller than those in the assay of pepsinogen I. However, the differences between the untreated plate and the plates of the present invention were still evident. Namely, for the untreated plates the residual content at 43°C was only 50.4%. However, for the PB 6.0-solidified plates and the HEPES 6.5-solidified plates, the residual contents at 43°C were 92.8% and 90.9%, respectively. Thus, also in the pepsinogen II assay, when the plates of the present invention were used, the stabilities of the measured values against the temperature burden were clearly better.

### Example 3: Assay of Prostate-Specific Acid Phosphatase (PAP) in the Serum Specimens Stored in pH Buffer Agent-Solidified Plates

PB 6.0-solidified plates were prepared in the same manner as described in Example 2. Serum specimen was prepared in the same manner as described in Example 1.

One hundred microliter per well of the serum specimen were poured into wells of the PB 6.0-solidified plates and untreated plates as controls. The plates were left for four hours under the temperature conditions of 4°C (control) and 37°C. The serum specimens were individually recovered and frozen at once.
Thereafter, the amounts of serum PAP were assayed using PAP RIA kit (DPC· PAP-IRMA kit, Diagnostic Products Corporation) according to the method recommended by the manufacturer. The results are shown in Table 4.

**Table 4**

| Container | Temp. Burden | | Residual Content (vs. 4°C) | | Residual Content (vs. untreated, 4°C) | |
|---|---|---|---|---|---|---|
| | None (4°C) | 37°C, 4hrs | None (4°C) | 37°C, 4hrs | None (4°C) | 37°C, 4hrs |
| Untreated Plate | 2.2 ng/ml | 0.3ng/ml | 100.0% | 13.6% | 100% | 13.6% |
| PB 6.0 solidified Plate | 2.3ng/ml | 2.1 ng/ml | 100.0% | 91.3% | 104.5% | 95.5% |

Standard value: 3.5ng/ml or less; Measurement Method: RIA (IRMA)

It is known that PAP rapidly loses its activity when it is left at 37 °C. For example, there are data that after incubating serum at 37 °C for four hours, the PAP activity (by an enzyme quantitative analysis) was reduced to 0% and the protein amount of PAP (by RIA) was reduced to about 30%, as compared to those at the starting point (0 hour).

In this experiment (i.e., Example 3), it has been shown that when a serum specimen had been stored in an untreated container at 37°C for four hours, the amount of PAP was reduced to 13.6% as compared to that stored in the same container at 4°C, and that when the same serum specimen had been stored in a container of the present invention (i.e., a PB 6.0-solidified plate) at 37°C for four hours, PAP was maintained in an amount of 91.3% as compared to that stored in the same container at 4°C and in an amount of 95.5% as compared to that stored in the untreated container at 4°C. Thus, the method and container of the present invention are very effective for stabilizing PAP.

The present invention is defined or limited only by the following claims.

## Claims

1. A method for stabilizing a blood, serum, or plasma specimen comprising:
1-a) adding a pH buffer agent to a blood specimen,
1-b) preparing a serum or plasma specimen and adding a pH buffer agent to the specimen, or
1-c) putting a blood, serum, or plasma specimen into a container that contains a pH buffer agent, and
2) storing the specimen obtained in step 1-a, 1-b, or 1-c,
wherein the pH buffer agent has an initial pH in the range of a weakly acidic pH to a slightly basic pH and has a buffer ability that maintains the pH of the specimen obtained in step 1-a, 1-b, or 1-c around neutral in step 2.

2. The method according to claim 1, wherein the pH buffer agent maintains the pH of the specimen within the range of pH 7.0 to pH 8.0 in step 2.

3. The method according to claim 1, wherein the pH buffer agent has an initial pH within the range of pH 5.5 to pH 8.0.

4. The method according to claim 1, wherein the pH buffer agent has a form selected from the group consisting of a solid, powder, granules, coating, a droplet, and microdroplets.

5. The method according to claim 1, wherein the pH buffer agent has been prepared by vacuum drying (including drying under reduced pressures and freeze drying), heat transfer drying (including convective heat transfer drying, radiation heat transfer drying, and conduction heat transfer drying), internal exothermic drying, drying by using a desiccant, ultrasonic drying, or air drying an aqueous solution of the pH buffer agent that has been contained in the container.

6. The method according to claim 1, wherein the container is a vacuum or non-vacuum blood collection tube, a centrifuge tube for separating serum or plasma, a urine collection cup, a container for storing a specimen, a 96-well microtiter plate or a module thereof, a 384-well plate, or a specimen container that is used when the specimen is sent to a clinical reference laboratory by mail.

7. A container that a blood, serum, or plasma specimen contacts, containing a pH buffer agent having an initial pH in the range of a weakly acidic pH to a slightly basic pH and a buffer ability that maintains the pH of the specimen around neutral.

8. The container according to claim 7, wherein the pH buffer agent maintains the pH of the specimen within the range of pH 7.0 to pH 8.0.

9. The container according to claim 7, wherein the pH buffer agent has an initial pH within the range of pH 5.5 to pH 8.0.

10. The container according to claim 7, wherein the pH buffer agent has a form selected from the group consisting of a solid, powder, granules, coating, a droplet, and microdroplets.

11. The container according to claim 7, wherein the pH buffer agent has been prepared by vacuum drying (including drying under reduced pressures and freeze drying), heat transfer drying (including convective heat transfer drying, radiation heat transfer drying, and conduction heat transfer drying), internal exothermic drying, drying by using a desiccant, ultrasonic drying, or air drying an aqueous solution of the pH buffer agent that has been contained in the container.

12. The container according to claim 7, wherein the container is a vacuum or non-vacuum blood collection tube, a centrifuge tube for separating serum or plasma, a urine collection cup, a container for storing a specimen, a 96-well microtiter plate or a module thereof, a 384-well plate, or a specimen container that is used when the specimen is sent to a clinical reference laboratory by mail.

13. A method for stabilizing a serum or plasma specimen comprising:
1-a) adding a pH buffer agent to a blood specimen, or
1-b) putting a blood specimen into a container that contains a pH buffer agent,
2) dissolving the pH buffer agent in the blood specimen,
3) preparing a serum or plasma specimen from the specimen obtained in step 2, and
4) storing the serum or plasma specimen obtained in step 3,
wherein the pH buffer agent has an initial pH in the range of a weakly acidic pH to a slightly basic pH and has a buffer ability that maintains the pH of the serum or plasma specimen obtained in step 3 around neutral in step 4.
